Europäisches Patentamt

European Patent Office

Office européen des brevets

(11) Veröffentlichungsnummer: **0 122 584**
**A2**

# (12) EUROPÄISCHE PATENTANMELDUNG

(21) Anmeldenummer: **84103976.1**

(22) Anmeldetag: **10.04.84**

(51) Int. Cl.³: **C 07 D 501/46**
**A 61 K 31/545**

(30) Priorität: **16.04.83 DE 3313816**

(43) Veröffentlichungstag der Anmeldung:
**24.10.84 Patentblatt 84/43**

(84) Benannte Vertragsstaaten:
**AT BE CH DE FR GB IT LI LU NL SE**

(71) Anmelder: **HOECHST AKTIENGESELLSCHAFT
Postfach 80 03 20
D-6230 Frankfurt am Main 80(DE)**

(72) Erfinder: **Dürckheimer, Walter, Dr.
Im Lerchenfeld 45
D-6234 Hattersheim am Main(DE)**

(54) **Neue Kristallmodifikation von Ceftazidim.**

(57) Kristallisiertes (6R, 7R)-7-[(Z) -2- (2-Aminothiazol-4-yl) -2- (2-carboxy-prop-2- oximino)- acetamido] -3-(1-pyridinium-methyl)- ceph-3-em-carboxylat x 1,5 Mol Wasser und Verfahren zur seiner Herstellung, gegen bakterielle Infektionen wirksame pharmazeutische Zubereitung, die diese Verbindung enthält sowie ein Verfahren zur Herstellung der Zubereitung und die Verwendung dieser Verbindung zur Bekämpfung bakterieller Infektionen.

EP 0 122 584 A2

0122584

HOECHST AKTIENGESELLSCHAFT    HOE 83/F 064  Dr. KA/Fr

Neue Kristallmodifikation von Ceftazidim

Das (6R,7R)-7-[(Z)-2-(2-Aminothiazol-4-yl)-2-(2-carboxy-prop-2-oximino)acetamido]-3-(1-pyridiniummethyl)-ceph-3-em-carboxylat (Ceftazidim) ist ein Cephalosporin-Antibiotikum der 3. Generation mit einer ausgeprägten Wirksamkeit gegenüber gram-negativen Erregern, einschließlich der Pseudomonas-Species. Das Pentahydrat dieser Verbindung (vgl. DE-OS 30 37 102) ist bisher die einzige kristalline Form, die für die parenterale Anwendung gut geeignet ist.

Es ist bekannt, daß Cephalosporine in ihrer Haltbarkeit begrenzt sind und sich beim Lagern leicht zersetzen. Ursache dafür ist der sehr hydrolyseempfindliche ß-Lactamring, der selbst mit kristallgitter gebundenem Wasser noch langsam reagieren kann, wenn das Molekül einer Temperaturbelastung ausgesetzt wird oder bei Raumtemperatur über längere Zeit gelagert wird. Es ist deshalb von großer Bedeutung, eine möglichst stabile Kristallform für die medizinische Anwendung eines Cephalosporin-Antibiotikums zu verwenden.

Es wurde nun gefunden, daß Ceftazidim in eine neue kristalline Modifikation überführt werden kann, die sich gegenüber dem Pentahydrat durch erhöhte thermische Stabilität auszeichnet und für eine parenterale Zubereitung sehr gut geeignet ist.

Gegenstand der Erfindung ist daher ein kristallines Ceftazidim x 1,5 Mol Wasser, seine pharmazeutischen Zubereitungen, sowie ein Verfahren zu seiner Herstellung, das dadurch gekennzeichnet ist, daß man wasserfreies, kristallines Ceftazidim an feuchter Luft bis zur Aufnahme der angegebenen Wassermenge rehydratisiert.

Die erfindungsgemäße Kristallmodifikation besitzt ein charakteristisches Deb ye-Scherrer-Diagramm (Tabelle 1) das das Vorliegen einer neuen Kristallform mit neuen physikalischen und chemischen Eigenschaften belegt.

Der Wassergehalt der erfindungsgemäßen Ceftazidim-Kristallmodifikation kann je nach Luftfeuchtigkeit, bei der die Proben gehandhabt und untersucht werden, um 0,2 bis 0,4 Mol schwanken, ohne daß eine Änderung der Kristallmodifikation beobachtet wird.

Die als Ausgangsmaterial einsetzbare wasserfreie, kristalline Form ist gemäß Patentanmeldung HOE 83 /F 063 (Patent .......... )) durch Dehydratisierung eines kristallinen Ceftazidim-Hydrats, vorzugsweise des Pentahydrats, bis zur Gewichtskonstanz erhältlich.

Die Wasseraufnahme kann mit Luft unter normalen Raumbedingungen oder mit erhöhtem Feuchtigkeitsgehalt, wie z.B. 50 bis 80 % relativer Luftfeuchtigkeit, durchgeführt werden. Der Feuchtigkeitsgehalt der Luft kann auch künstlich in bekannter Weise erhöht werden, z.B. durch vorheriges Mischen mit Wasserdampf.

Die gewünschte Menge an Kristallwasser wird durch Gewichtskontrolle ermittelt und nach Erreichen der vollständigen Kristallumwandlung in ein Hydrat mit 1,5 Mol Wasser beendet.

Die Rehydratisierung wird in der Regel bei Raumtemperatur vorgenommen, kann jeoch auch bei erhöhter (z.B. 40°C) oder niedrigerer Temperatur (z.B. 10°C) durchgeführt werden.

Die Zeit für die Dehydratisierung hängt insbesondere von der Teilchengröße des Ausgangsmaterials, der Schichthöhe des Materials und den äußeren Bedingungen, wie z.B.

Luftfeuchtigkeit und Temperatur ab. Sie kann so beispielsweise zwischen mehreren Stunden und Tagen liegen.

Die erfindungsgemäße Kristallmodifikation zeigt bei
Lagerung der Proben unter Temperaturbelastung im Vergleich zu Ceftazidim-Pentahydrat eine höhere Stabilität
und ist deshalb für die medizinische Anwendung besser
geeignet.

Die erfindungsgemäße Verbindung ist ein wertvolles
Antibiotikum, das zur Bekämpfung gram-positiver und vor
allem gram-negativer Infektionen geeignet ist (vgl.
DE-OS 29 21 316).

Gegenstand der Erfindung sind daher auch pharmazeutische
Zubereitungen, die die erfindungsgemäße Verbindung enthalten, wie z.B. Lösungen, Suspensionen oder Emulsionen
in öligen oder wässrigen Trägern.

Die erfindungsgemäße Verbindung kann als solche oder
zusammen mit therapeutisch üblicherweise eingesetzten
Hilfsstoffen, wie z.B. Formulierungs-, Lösungs-,
Suspendier- und/oder Dispergiermitteln kombiniert werden.
Der Wirkstoff kann in einer Zubereitung beispielsweise
auch vor seiner Verwendung in Pulverform zur Auflösung
in beispielsweise sterilem, pyrogenfreiem Wasser vorliegen. Zum Herstellen von wässrigen Lösungen wird der
Wirkstoff zweckmäßigerweise durch Zusatz eines basischen
Hilfsstoffes, wie z.B. Natriumcarbonat, Natriumbicarbonat,
Kaliumcarbonat, Kaliumbicarbonat, Lithiumcarbonat,
Calciumcarbonat, Magnesiumcarbonat, Trihydroxymethylmethylamin, Ethylendiamin, Lysin, Arginin, Glucosamin,
N-Methyl-glucosamin, Trihydroxymethyl-ethylamin, Diethanolamin, Diethylamin, Piperazin oder Procain gelöst.

Die Herstellung der Zubereitungen erfolgt in an sich
bekannter Weise beispielsweise durch Mischen, Einrühren, Lösen usw. mit/in den pharmazeutischen Hilfsstoffen.

Die Dosierung kann in einer Einheitsdosis beispielsweise zwischen etwa 50 und 1.500 mg Wirkstoff liegen, während eine Tagesdosis etwa 0,5 bis 6, vorzugsweise 1 bis 3 g betragen kann.

Beispiel

8,2 g kristallines und wasserfreies Caftazidim (erhalten nach Patentanmeldung                - HOE 83 / 063 (Patent ...........)) läßt man zwei Tage an der Luft stehen. Die relative Luftfeuchtigkeit lag bei der Versuchsdurchführung zwischen 50 und 70%. Nach dem angegebenen Zeitraum werden ca. 400 mg Wasser aufgenommen. Es entsteht quantitativ ein farbloses, kristallisiertes Ceftazidim mit 1,5 Mol Wasser, das sich im Deb ye-Scherrer-Diagramm (vergl. Tabelle 1) sehr charakteristisch von der wasserfreien Form und dem Pentahydrat von Ceftazidim unterscheidet. Geringe Schwankungen des Wassergehaltes beeinflussen die Struktur und Qualität der Kristalle nicht.

$C_{22}H_{22}N_6O_7S_2$ x 1,5 $H_2O$ (573,62)

|          | C     | H    | N     | S     | H    |
|----------|-------|------|-------|-------|------|
| berechnet: | 46,07 | 4,39 | 14,65 | 11,18 | 4,70 |
| gefunden   | 46,1  | 4,3  | 14,6  | 11,7  | 4,4  |

Das NMR-Spektrum in $CF_3CO_2D$ entspricht bezüglich der C-H-Protonenverschiebung vollständig dem des Pentahydrats.

## Tabelle 1

Charakteristische Kristallbeugungswinkel von Ceftazidim x 1,5 Mol $H_2O$

| Beugungswinkel $°2\vartheta$ (Cu-K$_\alpha$) | d $[Å°]$ | rel. Int. $[\%]$ |
|---|---|---|
| 6,75 | 13,1 | 10 |
| 8,80 | 10,4 | 100 |
| 11,7 | 7,56 | 10 |
| 13,0 | 6,80 | 25 |
| 14,25 | 6,21 | 15 |
| 16,25 | 5,45 | 20 |
| 16,60 | 5,34 | 25 |
| 18,50 | 4,79 | 25 |
| 18,90 | 4,69 | 20 |
| 19,40 | 4,57 | 55 |
| 20,80 | 4,27 | 20 |
| 21,15 | 4,20 | 30 |
| 21,90 | 4,05 | 30 |
| 23,25 | 3,82 | 10 |
| 24,70 | 3,60 | 15 |
| 25,20 | 3,53 | 20 |
| 26,30 | 3,38 | 10 |
| 26,50 | 3,36 | 10 |
| 27,10 | 3,29 | 10 |
| 28,25 | 3,16 | 10 |

Patentansprüche:

1. Kristallisiertes (6R,7R)-7-/(Z)-2-(2-Aminothiazol-4-yl)-2-(2-carboxy-prop-2-oximino)acetamido7-3-(1-pyridiniummethyl)-ceph-3-em-carboxylat x 1,5 Mol Wasser.

2. Verfahren zur Herstellung von kristallisiertem (6R,7R)-7-/(Z)-2-(2-Aminothiazol-4-yl)-2-(2-carboxyprop-2-oximino)acetamido7-3-(1-pyridiniummethyl)-ceph-3-em-carboxylat x 1,5 Mol Wasser, dadurch gekennzeichnet, daß man wasserfreies, kristallisiertes (6R,7R)-7-/(Z)-2-(2-Aminothiazol-4-yl)-2-(2-carboxyprop-2-oximino7acet-amido/-3-(1-pyridiniummethyl)-ceph-3-em-carboxylat an feuchter Luft bis zur Aufnahme der angegebenen Wassermenge rehydratisiert.

3. Gegen bakterielle Infektionen wirksame pharmazeutische Zubereitungen, gekennzeichnet durch einen Gehalt an kristallinem (6R,7R)-7-/(Z)-2-(2-Aminothiazol-4-yl)-2-(2-carboxyprop-2-oximino)acetamido7-3-(1-pyridinium-methyl)-ceph-3-em-carboxylat x 1,5 Mol Wasser.

4. Verfahren zur Herstellung von gegen bakterielle Infektionen wirksamen pharmazeutischen Präparaten, dadurch gekennzeichnet, daß das kristallisierte (6R,7R)-7-/(Z)-2-(Aminothiazol-4-yl)-2-(2-carboxy-prop-2-oximino)acetamido7-3-(1-pyridiniummethyl)-ceph-3-em-carboxylat x 1,5 Mol Wasser gegebenenfalls mit pharmazeutisch üblichen Hilfsstoffen in eine pharmazeutisch geeignete Verabreichungsform gebracht wird.

5. Verwendung von kristallisiertem (6R,7R)-/(Z)-2-(2-Aminothiazol-4-yl)-2-carboxyprop-2-oximino)acetamido7-3-(1-pyridiniummethyl)-ceph-3-em-carboxylat x 1,5 Mol Wasser zur Bekämpfung bakterieller Infektionen.

<u>Patentanspruch für Österreich:</u>

Verfahren zur Herstellung von kristallisiertem (6R,7R)-7-/(Z)-2-(2-Aminothiazol-4-yl)-2-(2-carboxyprop-2-oximino)acetamido/-3-(1-pyridiniummethyl)-ceph-3-em-carboxylat x 1,5 Mol Wasser, dadurch gekennzeichnet; daß man wasserfreies, kristallisiertes (6R,7R)-7-/(Z)-2-(2-Aminothiazol-4-yl)-2-(2-carboxyprop-2-oximino/acetamido/-3-(1-pyridiniummethyl)-ceph-3-em-carboxylat an feuchter Luft bis zur Aufnahme der angegebenen Wassermenge rehydratisiert.